# EUROPEAN PATENT APPLICATION

(11) **EP 4 506 355 A1**
(43) Date of publication of application: **12.02.2025**
(21) Application number: 23773969.3
(22) Date of filing: 23.03.2023
(51) Int. Cl.: C07J 9/00

(54) **METHOD FOR SYNTHESIZING 7-KETOLITHOCHOLIC ACID INTERMEDIATE AND USE THEREOF**

(30) Priority: 25.03.2022 CN 202210321879
(71) Applicant: Suzhou Entech New-Material Technology Co., Ltd, Suzhou, Jiangsu 215004 (CN)
(72) Inventor: YANG, Yingquan, Suzhou, Jiangsu 215004 (CN); YUAN, Haitao, Suzhou, Jiangsu 215004 (CN); ZHOU, Panlong, Suzhou, Jiangsu 215004 (CN); WANG, Fei, Suzhou, Jiangsu 215004 (CN); WU, Hucheng, Suzhou, Jiangsu 215004 (CN)
(74) Representative: Gille Hrabal Partnerschaftsgesellschaft mbB Patentanwälte
(86) International application number: PCT/CN2023/083444
(87) International publication number: WO 2023/179724

(57) **Abstract**

Provided in the present invention is a method for synthesizing a 7-ketolithocholic acid intermediate, the method comprising preparing compound OB-1 in an amide solvent to obtain a 7-ketolithocholic acid intermediate OB. The method of the present invention has the advantages of readily available of raw materials, a high yield, simple and mild reaction conditions, and suitability for industrial production.

## Description

The present invention claims the priority of the prior application with Patent Application No. 202210321879.4, entitled "Method for Synthesizing 7-Ketolithocholic Acid Intermediate and Use thereof" filed on March 25, 2022 to China National Intellectual Property Administration, the content of which is incorporated by reference herein in its entirety as part of the present invention.

### TECHNICAL FIELD

The present invention belongs to the field of pharmaceutical synthesis and specifically relates to a method for synthesizing a 7-ketolithocholic acid intermediate and use thereof.

### BACKGROUND

7-ketolithocholic acid has the CAS number of 4651-67-6, the molecular formula of C₂₄H₃₈O₄, the molecular weight of 390.56, and the structural formula as shown below: 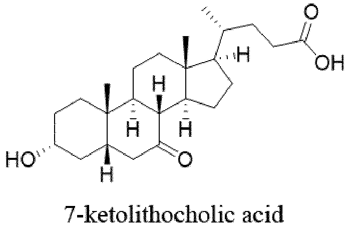 7-ketolithocholic acid is an important drug intermediate. 7-ketolithocholic acid may be used as an intermediate to synthesize chenodeoxycholic acid (Tetrahedron Letters Volume 24, Issue 24, 1983, Pages 2487-2490), ursodeoxycholic acid (J. Org. Chem. 1993, 58, 499-501), obeticholic acid (J. Med. Chem. 2002, 45, 17, 3569-3572) and the like.

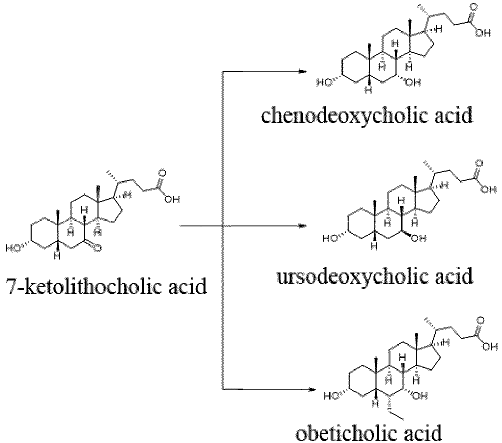

At present, most of the production processes for ursodeoxycholic acid and obeticholic acid are basically based on the method using 7-ketolithocholic acid as a raw material. Therefore, it becomes very important to effectively obtain 7-ketolithocholic acid.

Currently, 7-ketolithocholic acid is mainly synthesized by the following methods.

### I. Using cholic acid as the raw material

WO2014020024A1 reports that cholic acid is used as a raw material, the carboxy group on the side chain is esterified by methanol and hydrochloric acid, hydroxyl groups at both 3-position and 7-position are protected by acetic anhydride, hydroxyl at 12-position is oxidized by sodium hypochlorite to a carbonyl, ketone at 12-position is subjected to Wolff-Kishner-Huang-Minlon reduction, and finally, hydroxyl at 7-position is selectively oxidized by sodium hypobromite to ketone so as to obtain a target compound, namely 7-ketolithocholic acid. During the whole process, it is required to use the Wolff-Kishner-Huang-Minlon reaction performed at very high temperature. The reaction is performed at a relatively high temperature and hydrazine hydrate is highly toxic and explosive. Therefore, the requirements for equipment are relatively high.

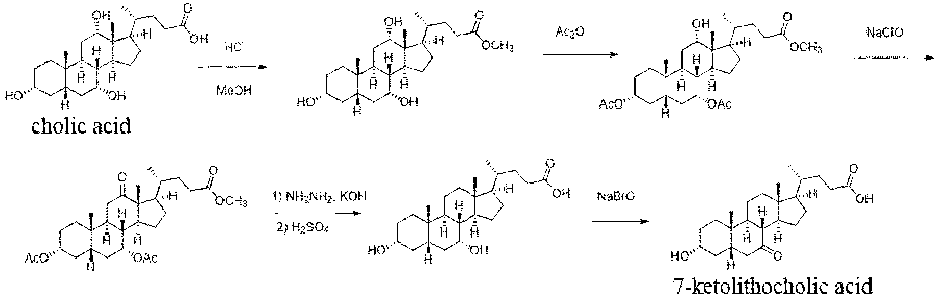

### II. Using chenodeoxycholic acid as the raw material

CN106046095 reports a method for obtaining 7-ketolithocholic acid by NBS oxidation of chenodeoxycholic acid in acetone and water. However, the chenodeoxycholic acid is expensive and use thereof is limited.

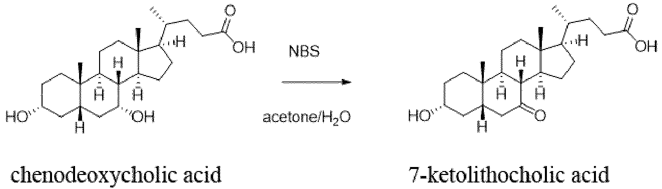

### III. Using hyocholic acid as the raw material

A patent publication CN110423261A reports a preparation method using hyocholic acid as a raw material. The disadvantages of the scheme are that: 1) chromium reagents such as Jones reagent are used, resulting in more pollution and environmental protection pressure; 2) a part of the reagents such as lithium iodide and TBSCl are expensive, and the whole cost of the route is high; and 3) pyridine is used as a solvent in 3^{rd} step, which has a strong odor and a high toxicity.

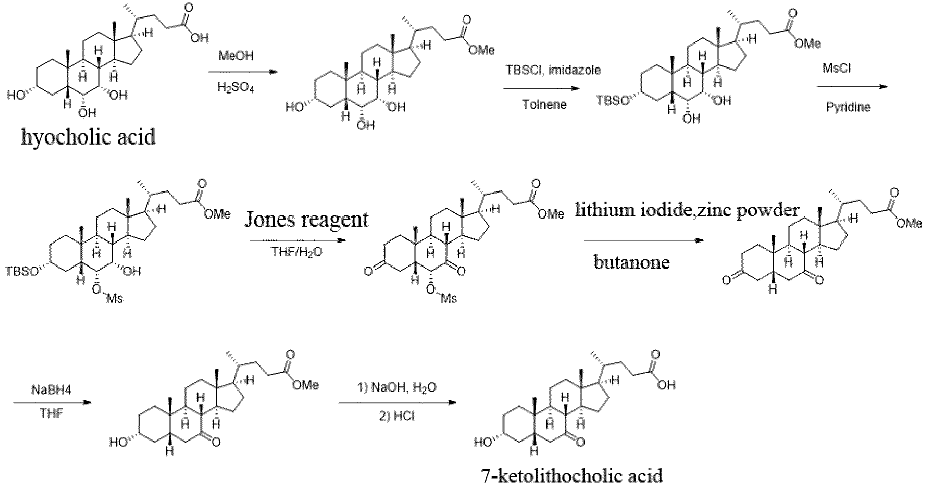

In addition to various disadvantages of the three methods mentioned above, another disadvantage is that the methods use animal-derived cholic acid. The animal body generally contains many animal viruses such as swine fever virus, avian influenza virus, prion and other various physiologically active substances, which have a certain biological toxicity to human body. Therefore, there is a need to find a new, safe and effective method for synthesizing a 7-ketolithocholic acid intermediate.

### SUMMARY

In order to ameliorate the above technical problems, the present invention provides a method for synthesizing a 7-ketolithocholic acid intermediate OB, comprising the following step of: performing a hydrogenation reaction on a compound OB-1 in an amide solvent to obtain the 7-ketolithocholic acid intermediate OB; 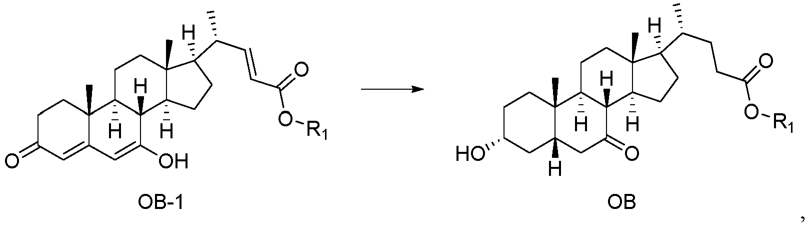 wherein R₁ is alkyl, for example C₁₋₆ alkyl, such as methyl, ethyl, propyl or tert-butyl.

According to an embodiment of the present invention, the reaction is performed in the presence of a catalyst, wherein the catalyst is for example selected from a Raney Ni catalyst, a Pd/C catalyst, a Pt/C catalyst or a Ru/C catalyst.

According to an embodiment of the present invention, the mass ratio of the OB-1 to the catalyst is (2-20): 1, for example (5-15): 1, exemplarily 10: 1.

According to an embodiment of the present invention, the amide solvent is, for example, selected from at least one of N,N-dimethylformamide, N,N-dimethylacetamide, formamide, N-methylpyrrolidone, N-methylformamide, N-methylacetamide and N,N-dimethylpropyleneurea.

According to an embodiment of the present invention, the mass-to-volume ratio of the OB-1 to the solvent in g:mL is 1:(1-20), for example 1:(1-10), exemplarily 1:6.

The inventors have found that the OB-1 has a tautomer 1:

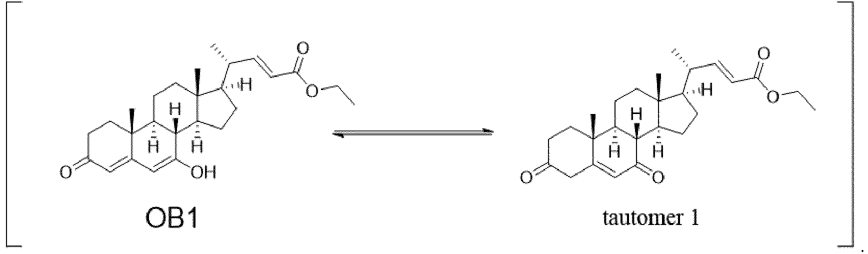

The amide solvent used in the present invention is weakly basic. In the presence of the amide solvent, the conversion of the tautomer to the OB-1 is favored, such that the product OB is mainly obtained under the conditions of the present invention.

According to an embodiment of the present invention, a method for preparing the compound OB-1 comprises the following steps of: 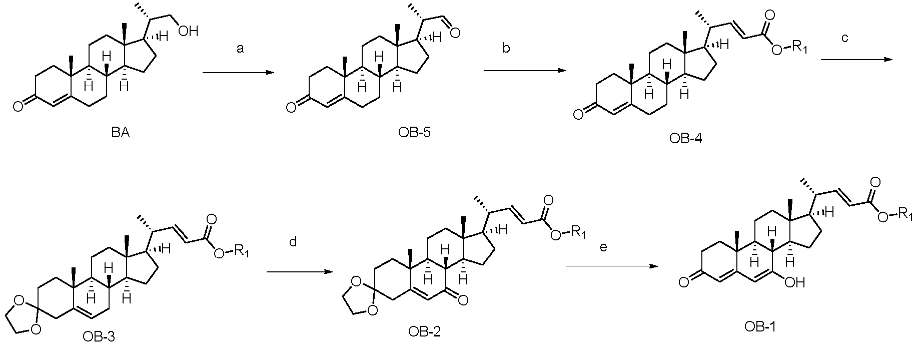 wherein R₁ is as defined above;
a) subjecting a compound BA to an oxidation reaction to obtain a compound OB-5;
b) subjecting the compound OB-5 and 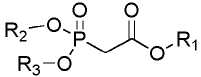 (R₂ and R₃ are C₁₋₆ alkyl, for example, ethyl), for example, triethyl phosphonoacetate ( 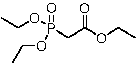 ) to a wittig reaction to obtain a compound OB-4; or subjecting the compound OB-5 and a compound 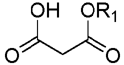 to a Knoevenagel condensation to obtain the compound OB-4;
c) subjecting the compound OB-4 to a protection of ethylene glycol to obtain a compound OB-3;
d) subjecting the compound OB-3 to an oxidation reaction to obtain a compound OB-2; and
e) subjecting the compound OB-2 to a de-protection of ethylene glycol to obtain the compound OB-1.

The present invention further provides a method for preparing 7-ketolithocholic acid, comprising preparing a compound OB according to the above steps and hydrolyzing the compound OB to obtain 7-ketolithocholic acid, 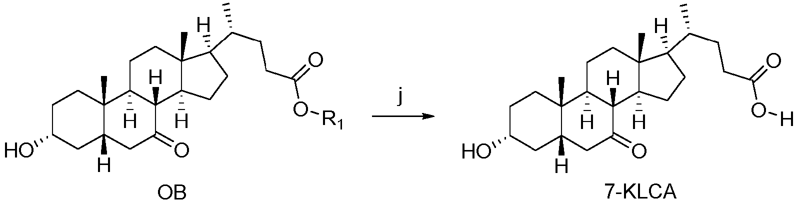 wherein R₁ is as defined above.

### Beneficial effects

The present invention provides a new method for synthesizing a 7-ketolithocholic acid intermediate OB, wherein a specific amide solvent is used to stabilize the structure of the raw material OB-1, so as to prepare the intermediate OB.

According to the present invention, bisnoralcohol (BA), a phytosterol biodegradable product, is used as a starting material, which is widely available, inexpensive, and is free of an animal virus. The raw material can be converted to the intermediate OB of 7-ketolithocholic acid by subjecting to oxidation reaction, wittig reaction, ketal protection, allylic oxidation reaction, ketal de-protection and hydrogenation reaction. The method of the present invention has the advantages of easily available raw materials, high yield, simple and mild reaction conditions and suitability for industrial production.

### Definition and description of terms

Unless otherwise indicated, the definitions of groups and terms described in the description and claims of the present application, including definitions thereof as examples, exemplary definitions, preferred definitions, definitions described in tables, definitions of specific compounds in the examples, etc., may be combined with each other at will. The definitions of the groups after such combination and structures of compounds should be understood to be within the scope described in the description and/or claims of the present application.

The term "C₁₋₆ alkyl" represents a straight or branched saturated monovalent hydrocarbyl having 1, 2, 3, 4, 5 or 6 carbon atoms. The alkyl is, for example, methyl, ethyl, n-propyl, n-butyl, n-pentyl, n-hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, etc., or isomers thereof.

### DETAILED DESCRIPTION

The technical solutions of the present invention will be further described in detail below in conjunction with specific examples. It should be understood that the following examples only exemplarily illustrate and explain the present invention and should not be construed as limiting the protection scope of the present invention. All the techniques realized on the basis of the above-mentioned contents of the present invention are covered in the protection scope of the present invention.

Unless otherwise indicated, the raw materials and reagents used in the following examples are all commercially-available, or may be prepared by known methods.

Compound 5 was prepared by a method of oxidizing BA with sodium hypochlorite in reference to patent application CN1137800A; and compound 4 was prepared from compound 5 through a wittig reaction with reference to Heletica Chimica Acta, 2002 vol.85, 4, p.1096-1101.

### Example 1 Synthesis of compound 3

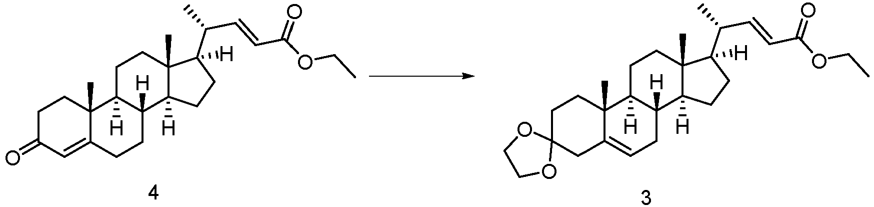

A compound 4 (50 g, 126 mmol), ethylene glycol (50 g, 806 mmol), DCM (600 mL), triethyl orthoformate (28 g, 189 mmol) and p-toluenesulfonic acid (0.5 g, 2.6 mmol) were added to a 1 L three-necked flask under protection of N₂, and stirred at 25°C for 10 h. When TLC showed that the reaction was complete, 1 mL of triethylamine was added and stirred for 30 min. The reaction solution was washed with 100 mL of water, subjected to liquid separation, dried with anhydrous sodium sulfate, and concentrated to remove the solvent to obtain a crude product, which was purified by column chromatography (n-hexane:EtOAc = 10:1) to obtain 51.1 g of compound 3 with the yield of 92% and purity of 95% by HPLC. ESI-MS[M+H]+ 443.35.

### Example 2 Synthesis of compound 2

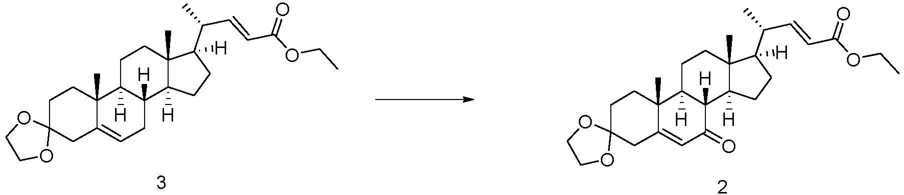

The compound 3 (44.2 g, 100 mmol), TBHP (120 mL, 600 mmol, 5.0 M n-decane solution), cuprous iodide (0.19 g, 1 mmol) and acetonitrile (300 mL) were added to a reaction flask, and reacted at 50 °C for 20 h. The reaction system was cooled to 25°C, and quenched by addition of aqueous saturated sodium sulfite solution (300 mL). The mixture was filtered through celite. The filtrate was extracted with ethyl acetate (200 mL×2). The organic phases were combined, dried over anhydrous sodium sulfate, filtered, concentrated under reduced pressure to remove the solvent, and purified by column chromatography (n-hexane:EtOAc = 6:1) to obtain 36.9 g of a product with the yield of 81% and the purity of 97% by HPLC. ESI-MS[M+H]+ 457.29.

### Example 3 Synthesis of compound 1

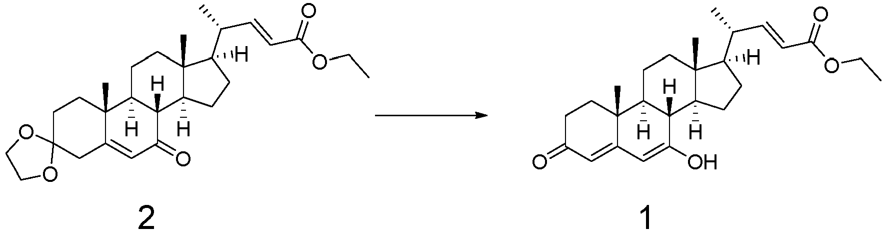

Water (40 mL) and THF (100 mL) were added to a 1 L single-neck flask, and concentrated sulfuric acid (4 g, 40.8 mmol) was added while stirring at 0°C. After stirring was performed for 10 min, compound 2 (12 g, 26.2 mmol) was added, the cold bath was removed, and stirring was performed at 25°C for 6 h. Water (100 mL) was added, and NaHCO₃ (10 g, 119 mmol) was added in batches to control the pH of 7-8. Then EtOAc (100 mL×2) was added for extraction. The organic phases were combined, dried over anhydrous sodium sulfate, filtered, and concentrated under reduced pressure to obtain a crude product as a yellow oil. The crude product was subjected to column chromatography (n-hexane:EtOAc = 3:1) to obtain 9.4 g of a solid as compound 1 with the yield of 87% and the purity of 94% by HPLC. ESI-MS[M+H]+ 413.37.

### Example 4 Synthesis of compound A

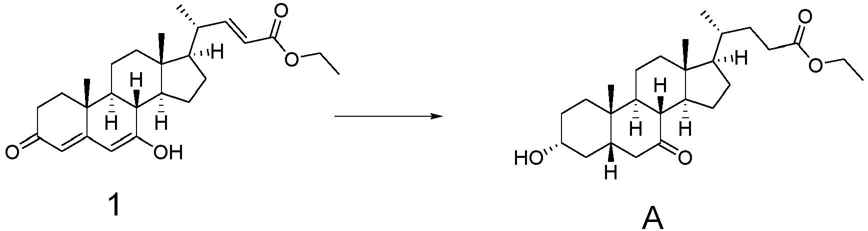

0.5 g of Raney nickel was added to the compound 1 (5.0 g, 12.13 mmol) in N,N-dimethylformamide (DMF) (30 mL) solution . The reaction mixture was hydrogenated at 25°C under 0.1 MPa of hydrogen for 12 h. After the hydrogenation was completed, the reaction solution was filtered and concentrated under reduced pressure to obtain 5.10 g of a crude product as compound A. The crude product was refined with acetone to obtain 4.57 g of a white solid A with the yield of 76% and the purity of 92% by HPLC. ESI-MS [M+H]⁺ 419.30.

¹H-NMR(DMSO-d6, 400MHz)δ(ppm): 4.48(d, J=4.8Hz, 1H), 4.04(q, J=6.8Hz, 2H), 2.90(dd, J=6Hz, 12Hz, 1H), 2.47-2.41(m, 1H), 2.36-2.27(m, 1H), 2.22-2.15(m, 1H), 2.09-2.02(m,1H), 1.94-1.90(m, 1H), 1.85-1.76(m, 2H), 1.73-1.64(m, 4H), 1.50-1.45(m, 2H), 1.39-1.30(m, 4H), 1.27-1.21(m, 2H), 1.17(t, J=6.8Hz, 3H), 1.14(s, 3H), 1.11-1.01(m, 5H), 0.96-0.77(m, 2H), 0.88(d, J=6.4Hz, 3H), 0.61(s, 3H).

### Example 5 Synthesis of compound A

0.5 g of 5% palladium carbon catalyst was added to the compound 1 (5.0 g, 12.13 mmol) in N,N-dimethylformamide (30 mL) solution. The reaction mixture was hydrogenated at 25°C under 0.1 MPa of hydrogen for 12 h. After the hydrogenation was completed, the reaction solution was filtered and concentrated under reduced pressure. The crude product was purified by column chromatography (n-hexane:EtOAc = 3:1) to obtain 3.8 g of a white solid A with the yield of 65% and the purity of 90% by HPLC. ESI-MS [M+H]⁺ 419.30.

### Example 6 Synthesis of compound A

0.5 g of 5% platinum carbon catalyst was added to the compound 1 (5.0 g, 12.13 mmol) in N,N-dimethylformamide (30 mL) solution. The reaction mixture was hydrogenated at 25°C under 0.1 MPa of hydrogen for 12 h. After the hydrogenation was completed, the reaction solution was filtered and concentrated under reduced pressure. The crude product was purified by column chromatography (n-hexane:EtOAc = 3:1) to obtain 3.19 g of a white solid A with the yield of 68% and the purity of 91% by HPLC. ESI-MS [M+H]⁺ 419.30.

### Example 7 Synthesis of compound A

0.5 g of 5% ruthenium carbon catalyst was added to the compound 1 (5.0 g, 12.13 mmol) in N,N-dimethylformamide (30 mL) solution. The reaction mixture was hydrogenated at 25°C under 0.1 MPa of hydrogen for 12 h. After the hydrogenation was completed, the reaction solution was filtered and concentrated under reduced pressure. The crude product was purified by column chromatography (n-hexane:EtOAc = 3:1) to obtain 3.45 g of a white solid A with the yield of 58% and the purity of 91% by HPLC. ESI-MS [M+H]⁺ 419.30.

### Example 8 Synthesis of compound A

0.5 g of Raney nickel was added to the compound 1 (5.0 g, 12.13 mmol) in N,N-dimethylacetamide (30 mL) solution. The reaction mixture was hydrogenated at 25°C under 0.1 MPa of hydrogen for 12 h. After the hydrogenation was completed, the reaction solution was filtered and concentrated under reduced pressure. The crude product was purified by column chromatography (n-hexane:EtOAc = 3:1) to obtain 4.46 g of a white solid A with the yield of 71% and the purity of 92% by HPLC. ESI-MS [M+H]⁺ 419.30.

### Example 9 Synthesis of compound A

0.5 g of Raney nickel was added to the compound 1 (5.0 g, 12.13 mmol) in formamide (30 mL) solution. The reaction mixture was hydrogenated at 25°C under 0.1 MPa of hydrogen for 12 h. After the hydrogenation was completed, the reaction solution was filtered and concentrated under reduced pressure. The crude product was purified by column chromatography (n-hexane:EtOAc = 3:1) to obtain 4.46 g of a white solid A with the yield of 67% and the purity of 92% by HPLC. ESI-MS[M+H]⁺ 419.30.

### Example 10 Synthesis of compound A

0.5 g of Raney nickel was added to the compound 1 (5.0 g, 12.13 mmol) in N-methylpyrrolidone (30 mL) solution. The reaction mixture was hydrogenated at 25°C under 0.1 MPa of hydrogen for 12 h. After the hydrogenation was completed, the reaction solution was filtered and concentrated under reduced pressure. The crude product was purified by column chromatography (n-hexane:EtOAc = 3:1) to obtain 4.31 g of a white solid with the yield of 62% and the purity of 91% by HPLC. ESI-MS [M+H]⁺ 419.30.

The foregoing exemplarily illustrates the implementation of the technical solutions of the present invention. It should be understood that the protection scope of the present invention is not limited to the above embodiments. Any modifications, equivalent replacements, improvements, and the like made by a person skilled in the art within the spirit and principle of the present invention shall fall within the protection scope of the claims of the present application.

## Claims

1. A method for synthesizing a 7-ketolithocholic acid intermediate OB, comprising the following step of: performing a hydrogenation reaction on a compound OB-1 in an amide solvent to obtain the 7-ketolithocholic acid intermediate OB; 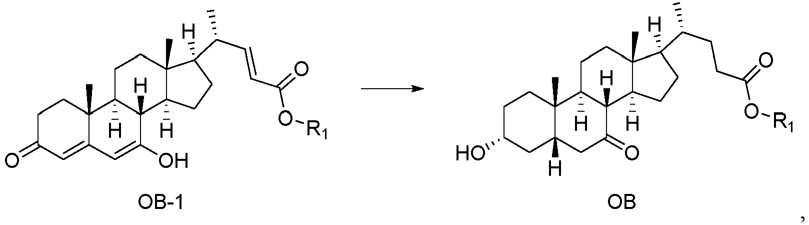 wherein R₁ is alkyl.

2. The method according to claim 1, wherein R₁ is C₁₋₆ alkyl, such as methyl, ethyl, n-propyl or tert-butyl.

3. The method according to claim 1 or 2, wherein the reaction is performed in the presence of a catalyst, wherein the catalyst is for example selected from a Raney Ni catalyst, a Pd/C catalyst, a Pt/C catalyst or a Ru/C catalyst.

4. The method according to claim 3, wherein the mass ratio of the OB-1 to the catalyst is (2-20):1.

5. The method according to any one of claims 1-4, wherein the amide solvent is selected from at least one of N,N-dimethylformamide, N,N-dimethylacetamide, formamide, N-methylpyrrolidone, N-methylformamide, N-methylacetamide and N,N-dimethylpropyleneurea.

6. The method according to any one of claims 1-5, wherein the mass-to-volume ratio of the OB1 to the solvent in g:mL is 1:(1-20).

7. The method according to any one of claims 1-6, wherein a method for preparing the compound OB-1 comprises the following steps of: 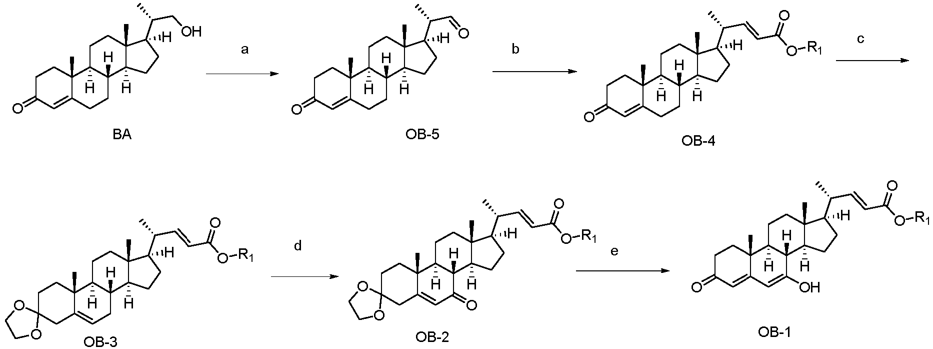 wherein R₁ is defined according to claim 1;
a) subjecting a compound BA to an oxidation reaction to obtain a compound OB-5;
b) subjecting the compound OB-5 and 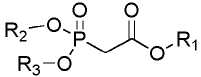 wherein R₂ and R₃ are C₁₋₆ alkyl, for example, triethyl phosphonoacetate ( 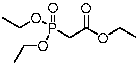 ) to a wittig reaction to obtain a compound OB-4; or subjecting the compound OB-5 and a compound 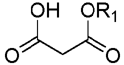 to a Knoevenagel condensation to obtain the compound OB-4;
c) subjecting the compound OB-4 to a protection of ethylene glycol to obtain a compound OB-3;
d) subjecting the compound OB-3 to an oxidation reaction to obtain a compound OB-2; and
e) subjecting the compound OB-2 to a de-protection of ethylene glycol to obtain the compound OB-1.

8. A method for preparing 7-ketolithocholic acid, comprising using the method according to any one of claims 1-7 to prepare the compound OB and hydrolyzing the compound OB to obtain 7-ketolithocholic acid, 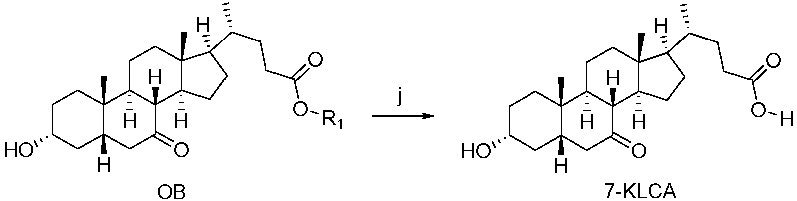 wherein R₁ is defined according to claim 1.
